# EUROPEAN PATENT SPECIFICATION

(11) **EP 0 942 750 B1**
(45) Date of publication and mention of the grant of the patent: **04.07.2007**
(21) Application number: 97941124.6
(22) Date of filing: 24.09.1997
(51) Int. Cl.: A61K 39/395, A61K 38/13, C07K 16/28, C07K 14/74

(54) **ANTI-CLASS II MHC BINDING AGENTS FOR USE IN XENOTRANSPLANTATION**
ANTI-MHC KLASSE II BINDUNGSREAGENTIEN ZUR VERWENDUNG BEI XENOTRANSPLANTATIONEN
AGENTS DE FIXATION ANTI-CLASSE II DU CMH, UTILISES DANS LES HETEROGREFFES

(30) Priority: 24.09.1996 GB 9619894
(43) Date of publication of application: 22.09.1999
(73) Proprietor: CELLTECH THERAPEUTICS LIMITED, Slough Berkshire SL1 4EN (GB)
(72) Inventor: SAXTON, Nina, Elizabeth, Taplow, Berkshire SL6 0DH (GB); FOULKES, Roland, Burnham, Buckinghamshire SL6 8AY (GB)
(74) Representative: Mercer, Christopher Paul
(86) International application number: PCT/GB1997/002599
(87) International publication number: WO 1998/013066

(56) References cited:
- WO-A-94/28897
- WO-A-96/38543
- PRUITT, SCOTT K. ET AL: "The effect of xenoreactive antibody and B cell depletion on hyperacute rejection of guinea pig-to-rat cardiac xenografts" TRANSPLANTATION (1993), 56(6), 1318-24 CODEN: TRPLAU;ISSN: 0041-1337, 1993, XP002051136

## Description

This invention relates to pharmaceutical products for the suppression or elimination of the rejection of xenografts and to the manufacture of such products.

One of the major factors influencing the number of solid organ allografts in human transplantation is the availability of suitable human donor organs. Once organs are available, modern surgical techniques and immunosuppressive therapy, although by no means ideal in terms of patient compliance, can then usually prolong survival. The shortage of suitable human organs however means that in many cases there is a long wait for a transplant. This wait may be fatal, particularly for patients awaiting a heart or liver transplant.

Transplantation of organs from non-human species (xenografting) would avoid the problem of human organ availability. Xenografting currently does not succeed however because of pre-existing anti-species antibodies in the human host which mediate hyperacute rejection. The rejection process is thought to be due in large part to a T-cell independent B cell effect, which in discordant grafting, (a transplant between distantly related species, e.g. pig to human) can result in a vigorous, complement-mediated destruction of the graft within minutes. With a concordant graft (a transplant between closely related species, e.g. baboon to human) the hyperacute response (sometimes called delayed acute rejection) can be less vigorous and occurs within a few days but is again due to T-cell independent B cell effects.

The B-cell effect has been illustrated in rats in which hamster-to-rat (concordant) heterotopic heart transplants reject in around 3 days. This is not prolonged by normal T-cell immunosuppressive treatment using cyclosporin A with doses which would normally prolong allograft survival indefinitely. However, if recipients are treated with cobra venom factor or cyclophosphamide to inhibit complement activity or lymphocyte proliferation this initial rejection phase can be overcome. T-cell immunosuppression can then be used [van den Bogaerde, J et al, Transplant Proc. 24, 513-514 (1992); Hasan, R. I. R. et al ibid, 24, 517-518 (1992)].

Recent progress using animals (e.g. pig) transgenic for human complement regulatory protein (e.g. DAF) has shown that many of the complement mediated effects can be overcome, for example in a pig to cynomolgus monkey xenograft. However, large doses of cyclophosphamide are still needed to suppress the T-cell independent B cell response. In a clinical situation this would not be acceptable due to the marked toxicity of cyclophosphamide. Ideally an agent which could reduce B cell responses during xenotransplantation, at least during the early phase and to a degree where an immunosuppressant such as cyclophosphamide either need not be administered or may be used at clinically acceptable doses, would be useful.

An alternative approach was described by Pruitt et al. (Transplantation, 1993, 56(6):1318-24) in which an antibody specific for rat class II MHC was administered to rats in an attempt to prevent xenograft rejection.

We have now surprisingly found that an antibody to a rat Class II major histocompatibility complex protein can inhibit B-cell function in *vivo* and is capable of prolonging the time to rejection of a xenograft in a hamster-to-rat xenograft model. Unexpectedly, when the antibody is used in conjunction with the immunosuppressant cyclosporin A in the same model the survival of the xenograft is markedly prolonged beyond the times when rejection occurs in the presence of one or other of the antibody or cyclosporin A alone. This has allowed us to develop a general means to suppress or eliminate rejection of a xenogeneic transplant which in particular avoids the use of potentially toxic doses of cyclophosphamide and similarly acting compounds.

Accordingly, the invention provides a pharmaceutical product comprising an anti-Class II major histocompatability complex antibody, or antigen binding fragment thereof and at least one other, different immunosuppressant for use to suppress or eliminate the rejection of a xenograft in a host.

In the specification hereinafter the abbreviation "MHC" is used to represent the term "major histocompatibility complex".

The term "xenograft" is used in conventional fashion to mean a graft derived from a donor species unrelated to the host species. The donor and host species may be for example any mammalian species and may be concordant, or closely related, e.g. chimpanzee or baboon donors and a human host; or discordant, or distantly related, e.g. a swine donor and a human host. The graft may be any organ or tissue (including isolated cells), and may be in particular a solid organ such as a heart, liver or kidney, or a tissue such as pancreatic tissue, e.g. pancreatic β-islet cells.

One such pharmaceutical product may take the form of a pharmaceutical composition in which the anti-Class II MHC antibody and the other immunosuppressant(s) are formulated in admixture, optionally together with a pharmaceutically acceptable excipient, diluent, or carrier and the invention extends to such compositions.

In the products according to the invention the Class II MHC protein binding antibody is one which recognises the Class II MHC protein and in so doing may inhibit any T-cell independent B-cell response. Except where otherwise indicated, an antibody of this type is hereinafter referred to as an anti-MHC II antibody.

Anti-MHC II antibodies are well-known in the art to interfere with T-cell activation [see for example Wraith, D. C. et al, Cell, 57, 709-715 (1989)]. On the basis of this they have been suggested previously for use in the treatment of immunological diseases, including transplant related conditions such as graft versus host disease and allograft rejection where it is important to suppress inappropriate T-cell activation (see for example International Patent Specification No. WO 94/29451). In the present invention, selection of the anti-MHC II antibody is based on its ability to inhibit T-cell independent B-cell function, and use is made of this during the delayed acute rejection phase of the xenograft. Once past the delayed acute rejection phase the continued presence of the antibody may also be beneficial in helping to suppress any T-cell mediated rejection of the graft.

The anti-MHC II antibody for use in the product according to the invention may be a whole antibody or an antigen binding fragment thereof, for example a Fab fragment or, especially, a bivalent fragment, such as a F(ab')₂ fragment. The antibody may be of animal, for example mammalian origin and may be for example of murine, rat or human origin. It may be polyspecific, but is preferably monospecific for a MHC II protein, especially a human MHC II protein. The antibody will in particular be one which is capable of inhibiting a T-cell independent B-cell response. It may be a polyclonal antibody or, preferably, a monoclonal antibody. Where desired, it may be a labelled antibody, the label being for example a reporter or effector group.

A number of anti-MHC II antibodies for use in the invention can be obtained from either freely or commercially available hybridoma cell lines, for example the cell lines ATCC accession No. HB55 and ATCC Accession No. HB151 [American Type Culture Collection, Rockville, Maryland, USA] producing the antibodies L243 and SFR3-DR5 respectively. Alternatively the antibody may be obtained using conventional immunisation and/or recombinant DNA techniques.

Thus, for example polyclonal antibodies may be obtained from the sera of animals immunised with a Class II MHC protein immunogen. Well known methods may be used to obtain the immunogen either from readily available cell sources or gene and/or protein sequence data [see for example Culley, D et al, Cell. lmmunol. 149, 279-290 (1993); Andersson, G et al, J. Biol. Chem. 262, 8748-8758 (1987); Servenius, B et al, ibid 262, 8759-8766 (1987), Jonsson, A-K et al, ibid 262, 8767-8777 (1987)]. Any suitable host, for example BALB/c mice where it is desired to obtain a mouse polyclonal antibody, may be injected with the immunogen, the serum collected and the antibody recovered therefrom. Monoclonal antibodies may be obtained from hybridomas derived from the spleen cells of an animal immunised as just discussed and fused to an appropriate "immortal" B-tumour cell. In each instance, the antibody may be recovered from either the serum or the hybridoma by making use of standard selection and subsequent purification and or concentration techniques, for example by chromatography, using for example Protein A or by other affinity chromatography. Selection of the antibody may be by any conventional selection means, for example by use of one or more cell based assay systems utilising appropriate indicator cell lines, for example as described in International Patent Specification No. WO 94/29451. In particular, part of the selection process will be based on the ability of the antibody to inhibit T-cell independent B-cell function.

Once a cell line, for example a hybridoma, expressing an antibody for use in the invention has been obtained it is possible to clone therefrom the cDNA and to identify the variable region genes encoding the desired antibody, including the sequences encoding the CDRs. From here, other recombinant antibodies for use in to the invention may be obtained by preparing one or more replicable expression vectors containing at least the DNA sequence encoding the variable domain of the antibody heavy or light chain and optionally other DNA sequences encoding remaining portions of the heavy and/or light chains as desired, and transforming an appropriate cell line, e.g. a non-producing myeloma cell line, such as a mouse NSO line, in which production of the antibody will occur. In order to obtain efficient transcription and translation, the DNA sequence in each vector should include appropriate regulatory sequences, particularly a promoter and leader sequence operably linked to the variable domain sequence. Particular methods for producing antibodies in this way are generally well known and routinely used. For example, basic molecular biology procedures are described by Maniatis et al [Molecular Cloning, Cold Spring Harbor Laboratory, New York, 1989]; DNA sequencing can be performed as described in Sanger et al [PNAS 74, 5463, (1977)] and the Amersham International plc sequencing handbook; and site directed mutagenesis can be carried out according to the method of Kramer et al [Nucl. Acids Res. 12, 9441, (1984)] and the Anglian Biotechnology Ltd handbook. Additionally, there are numerous publications, including patent specifications, detailing techniques suitable for the preparation of antibodies by manipulation of DNA, creation of expression vectors and transformation of appropriate cells, for example as reviewed by Mountain A and Adair, J R in Biotechnology and Genetic Engineering Reviews [ed. Tombs, M P, 10, Chapter 1, 1992, Intercept, Andover, UK] and in International Patent Specification No. WO 91/09967.

In the above aspects of the invention, and in those described hereinafter, the xenograft may be from a non-human species and the host is a human host. In these circumstances, when the Class II MHC protein binding agent is an anti-MHC II antibody, the antibody is preferably an anti-human MHC II antibody. Particular examples of such antibodies are the L243 and SFR3-DR5 antibodies and fragments thereof described above. In one particular preference the antibody is a recombinant version of one of these, particularly the L243 derived recombinant antibodies described in International Patent Specification No. WO 94/29451 and especially the antibody L243-gH/L243-gL1 described therein.

As explained above, and described in the Example hereinafter, the anti MHC II antibody is used in conjunction with at least one other immunosuppressant. One particular class of immunosuppressant for such a use according to the invention is a class containing agents capable of suppressing T-cell activation. Numerous examples of such agents are well known in the art and some are in routine use in the clinic, for example in the treatment of allograft rejection [see for example Morris, R. E. Transplantation Society Bulletin 1, 15-20 (1993)]. Particular examples include: (1) compounds which inhibit cytokine synthesis, for example the cyclosporins, e.g. cyclosporin A and analogues or derivatives thereof such as cyclosporin G or the hydroxyethyl derivative of D-serine cyclosporin A [see for example Burdmann, E. A et al, J. ASN 3, 841 (1992); and Hiestand, P. C. et al, Transplant. Proc. 25, 691-692 (1993)] or tacrolimus (FK506) or analogues or derivatives thereof [see for example Klintmalm, G.B. Transplant. Proc. 28, 974-976 (1996)]; (2) compounds which inhibit cytokine action, for example macrolide immunosuppressants such as rapamycin and analogues or derivatives thereof, or isoxazoles such as leflunomide and analogues or derivatives thereof; [see for example Thompson, A.W. Immunol. Lett. 29, 105-112 (1991); and Bartlett, R. R. et al, Agents Actions 32, 10-21 (1991)] (3) inhibitors of DNA synthesis, such as mizoribine, mycophenolic acid or brequinar; [see for example Cramer, D.V. et al Transplantation 53, 303 (1992); and Gray, D. W. R. Lancet 346, 390 (1995)] or (4) inhibitors of cell maturation such as deoxy-spergualin [see for example Ameniya, H et al, Transplant Proc. 23, 1087 (1991)].

If desired more than one immunosuppressant may be used in conjunction with the anti Class II MHC antibody in the products according to the invention. In addition the products may also contain other active ingredients, for example inhibitors of B-cell function such as cyclophosphamide. In each instance, as explained previously, the presence of the anti Class II MHC antibody allows, where desired, the other active ingredients to be used at doses where any unacceptable side-effects are reduced or avoided.

Formulation of the anti Class II MHC antibody and any other active ingredient for use in a product according to the invention may be carried out using conventional procedures. Each active ingredient may take any suitable form for administration to the host, for example a form for oral, parenteral or rectal administration.

Where the active ingredient is for parenteral administration, for example for intravenous, intramuscular or subcutaneous injection or infusion, it may be presented in unit dosage form, e.g. in glass ampoule or multi dose containers, e.g. glass vials. It may be formulated as a suspension, solution or emulsion in an oily or aqueous vehicle optionally containing formulatory agents such as suspending, stabilising, preserving and/or dispersing agents. Alternatively the active ingredient may be in a dry form, e.g. a powder for reconstitution before use with an appropriate sterile liquid, e.g. sterile pyrogen-free water.

For oral administration, the active ingredient may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.
For rectal administration the active ingredient may be formulated with a binding and/or lubricating agent, for example with a polymeric glycol, a gelatin, cocoa-butter or other vegetable wax or fat.

The active ingredient(s) may, if desired, be presented in a pack or dispenser device which may contain one or more unit dosage forms containing each active ingredient. The pack or dispensing device may be accompanied by instructions for administration.

Note that the product according to the invention contains an anti-MHC II antibody which is likely to be unsuitable for oral administration therefore the product is preferably used in a formulation for parenteral administration using, for example, one of the approaches described above.

According to a further aspect of the invention we provide the use of an anti Class II MHC antibody and at least one other, different immunosuppressant in the manufacture of a pharmaceutical product for use to suppress or eliminate the rejection of a xenograft in a host. Thus for example the anti Class II MHC antibody and the immunosuppressant(s) may be mixed together and other ingredients, e.g. a pharmaceutically acceptable excipient, diluent or carrier, also mixed in as required, to yield for example a product formulated for oral, parenteral or rectal administration as described previously.

The products according to the invention will contain active ingredients at a therapeutically effective dose and in a further aspect of the invention we provide a method of suppressing or eliminating the rejection of a xenograft in a host, the method comprising administering to the host a pharmaceutical product comprising an effective amount of a Class II MHC protein binding agent. In this aspect of the invention the pharmaceutical product may also contain an effective amount of one or more other, different immunosuppressants as described above.

Administration of the products according to the invention to the host generally will be before, during and/or after the transplant operation. In particular, however, to avoid the hyperacute rejection associated with the T-cell independent B-cell response it will be necessary to ensure that the product is present in sufficient quantity in the host at the beginning of the transplant procedure. In practice this may mean administering the product to the host prior to the transplant procedure for one or more days. In addition, it may be advantageous to ensure that the organ or tissue to be transplanted is contacted with the product prior to the transplant procedure, for example by bathing the isolated organ or tissue in a sterile solution containing the product. The administration of the product may be continued during or after the transplant operation for as long as the xenograft is at risk from an hyperacute rejection. The administration may then be discontinued as desired and reintroduced as necessary. Where the invention utilises other immunosuppressants these may generally be administered at the time of the transplant and thereafter on a continuous basis for as long as it is desired to suppress or eliminate any T-cell mediated rejection.

The doses at which the anti Class II MHC antibody and other immunosuppressant(s) will be administered will depend for example on the nature of the xenograft and other factors such as the age and condition of the host and the route of administration. In general the active ingredients will be used at doses generally recognised to be effective for the class of compound involved. Thus, for example the anti-MHC II antibody may be used at a dose between 0.1 - 100mg/Kg, e.g. 0.1 - 50mg/kg, preferably 0.1 - 10mg/kg body weight per single dose, depending on the nature, e.g. avidity of the antibody. Other immunosuppressants, for example the T-cell activation inhibitors described above, may be used at doses within the range 0.01 - 50mg/kg, preferably 0.05 - 20 mg/kg body weight per single dose. The doses may be administered singly one or more times a day or continuously during a day up to a maximum effective or tolerated total dose. Where appropriate initial daily doses may be varied once the hyperacute rejection phase has been passed, for example to reduce the frequency and/or quantity of dose.

### EXAMPLES

The following Examples illustrate the invention and demonstrate the effect of an anti-rat MHC II mouse monoclonal antibody (OX-6) in a hamster-to-rat xenograft model. The model used in each Example was as follows:

### Graft Survival Studies

Hearts from male hamsters were transplanted into the abdomens of rat (DA strain, RT1 ^{avl}) recipients and viability of each graft was assessed by external palpation on a grade of 0-4 (with 4 indicating a normally beating graft and 0 indicating rejection). The anti class II Mab used was OX-6 (anti-rat RT1-B monoclonal antibody; European Collection of Animal Cell Cultures, ECACC No. 84112007).

The hamster-to-rat xenograft provides a recognised model for xenogeneic grafting between other species and the results obtained would be expected to be found, for example, in non-human-to-human xenografts, for example swine-to-human.

### Example 1

### Effect of OX6 IgG alone or with Cyclosporin A (CsA)

DA rat recipients were treated as shown in Table 1

**TABLE 1**

| **Treatment** | **Time to rejection (days)** |
|---|---|
| Saline only | 3, 3, 3, 3, 3, 3 |
| OX6 (40mg/kg IP (day -1, 0, 1) | 5, 5, 5, 5, 5, 5 |
| CsA (30mg/kg PO daily from day 0) | 3, 3, 3, 3 |
| OX6 (40mg/kg day -1,0, +1) and CsA (30mg/kg day 0-7 then 20mg/kg every other day) | 9, 65, 65+, 73, 100+, 100+ |

| | |
|---|---|
| "+" denotes that the graft had not rejected at the time the animal was examined. | |

These data indicate that OX-6 IgG alone can prolong the time to rejection of a xenogeneic graft by 2 days and, when in combination with an immunosuppressive dose of cyclosporin A, can markedly increase the survival time beyond that of either agent alone.

### Example 2

### Effect of OX-6 F(ab')₂ alone or with cyclosporin A (CsA)

DA rat recipients were treated as shown in Table 2.

**TABLE 2**

| **Treatment** | **Time to rejection (days)** |
|---|---|
| Saline only | 3, 3, 3, 3, 3, 3 |
| OX6 F(ab')₂ (80mg/kg IP days -1, 0, +1) | 4, 4, 4, 5, 5, 5 |
| OX6 F(ab')₂ (80mg/kg IP, days -1, 0, +1) and CsA (30mg/kg days 0-7 then 20mg/kg every other day PO) | 11, 26, 27, 41+ 42+. 67, 67+, 95+, 95+ |

| | |
|---|---|
| "+" denotes that the graft had not rejected at the time the animal was examined. | |

These data indicate that OX-6 F(ab')₂ alone can prolong the time to rejection of a xenogeneic graft by 2 days and, when in combination with an immunosuppressive dose of cyclosporin A, can markedly increase the survival time beyond that of either agent alone.

### Example 3

### Effect of OX-6 IgG alone or with mycophenolic acid (MMF)

DA rat recipients were treated as shown in Table 3.

**TABLE 3**

| **Treatment** | **Time to rejection (days)** |
|---|---|
| Saline only | 3, 3, 3, 3, 3, 3 |
| OX6 IgG (40mg/kg IP days -1, 0, +1) | 5, 5, 5, 5, 5, 5 |
| MMF (40mg/kg PO daily) | 4, 5, 5, 5, 5, 5 |
| OX6 IgG (40mg/kg IP day -1, 0, +1) and MMF (40mg/kg daily) | 8, 8, 8, 8, 9, 9 |

These data indicate that OX-6 IgG in combination with an immunosuppressive dose of mycophenolic acid can increase graft survival time beyond that of either agent alone.

### Example 4

### Effect of OX6 IgG alone or with tacrolimus (FK506

DA rat recipients were treated as shown in Table 4.

**TABLE 4**

| **Treatment** | **Time to rejection (days)** |
|---|---|
| Saline only | 3, 3, 3, 3, 3, 3 |
| OX6 IgG (40mg/kg IP days -1, 0, +1) | 5, 5, 5, 5, 5, 5 |
| FK506 (1 mg/kg IP daily) | 3, 3, 3, 4, 4 |
| OX6 IgG (40mg/kg IP, days -1, 0, +1), and FK506 (1 mg/kg I P daily) | 6, 6, 6, 6, 7, 13+ |

| | |
|---|---|
| "+" denotes that the graft had not rejected at the time the animal was examined. | |

These data indicate that OX-6 IgG in combination with an immunosuppressive dose of FK506 can increase graft survival time beyond that of either agent alone.

### Example 5

### Inhibition of B-cell function

### Materials and Methods

### Anti-hamster lytic antibody assay

Transplanted DA rats (see 'Graft Survival Studies' above) were given either saline, OX68¹ or (Fab')₂ OX6 intraperitoneally at 40mg/kg and 80mg/kg, respectively, on days -1 and 0. Groups of rats were bled following surgery on days 1, 3, 5 and 7. The sera from these samples were assayed for anti-hamster titre as follows. In a 96 well round bottomed microtitre plate, 25µl of a 1% hamster erythrocyte suspension was added to 25µl of test serum that had been serially diluted in complement fixation diluent (CFD). To this 25µl of a 1:7 dilution of baby rabbit complement (Serotec, Oxford, UK) was added and incubated for 1.5h at 37°C. CFD (150µl) was added and the plate centrifuged at 150g for 4 minutes before 150µl of supernatant was transferred into an ELISA plate and the Absorbance read at 415nm wavelength. Positive and negative controls were distilled water or CFD, respectively.

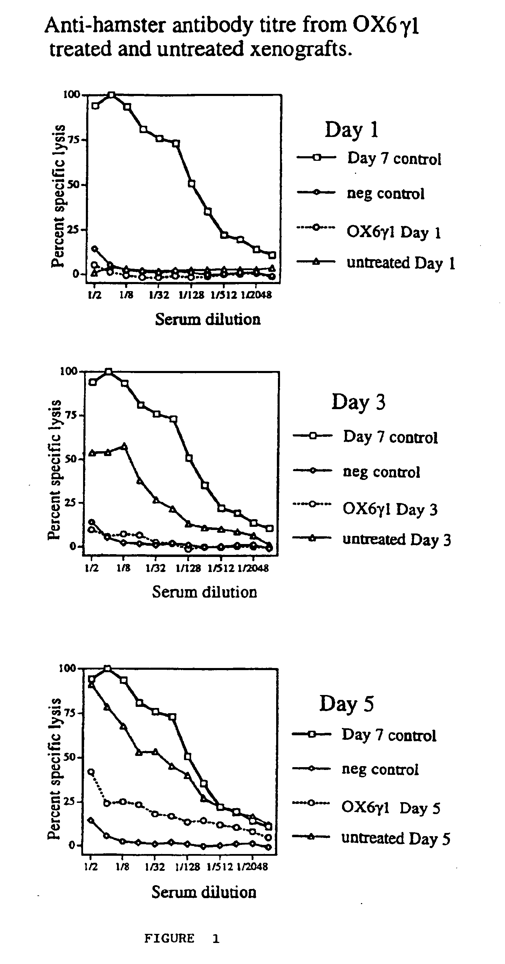

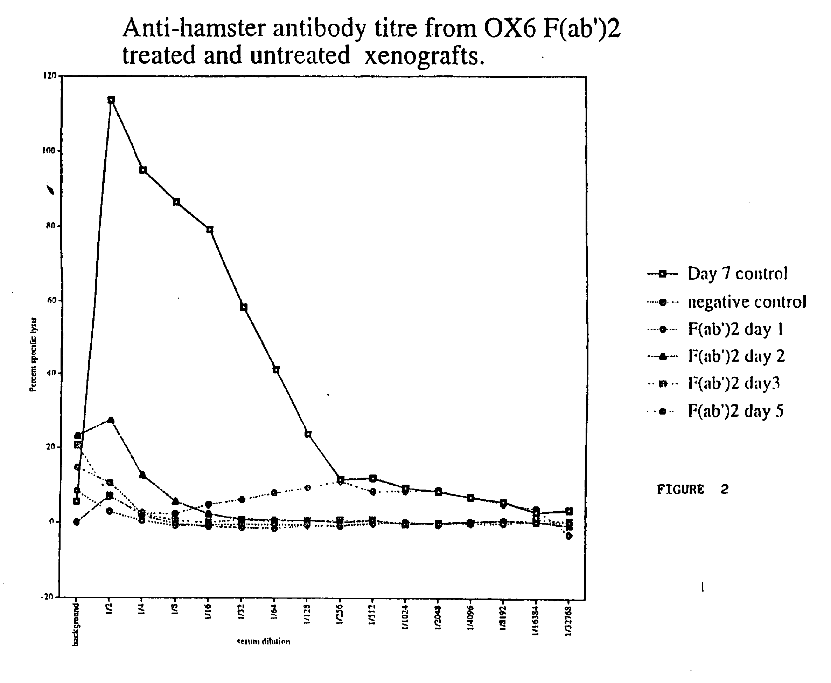

Figures 1 and 2 illustrate the results obtained in one experiment. The data indicates that OX6 IgG8¹ or F(ab')₂ can inhibit xenogeneic antibody production in DA rat recipients. This demonstrates that OX6 can inhibit B cell function *in vivo.*

## Claims

1. A pharmaceutical product comprising an anti-Class II major histocompatability complex antibody, or antigen binding fragment thereof and at least one other, different immunosuppressant for use to suppress or eliminate the rejection of a xenograft in a host.

2. A pharmaceutical product according to claim 1, wherein the anti-Class II MHC antibody, or antigen binding fragment thereof is capable of suppressing or eliminating a host T-cell independent B-cell response.

3. A pharmaceutical product according to claim 1 or claim 2, wherein the other immunosuppressant is capable of suppressing T-cell activation.

4. A pharmaceutical product according to any of claims I to 3, wherein the antibody is an anti-human Class II major histocompatability complex antibody, or an antigen binding fragment thereof.

5. A pharmaceutical product according to claim 4, wherein the antibody is L243, SFR3-DR5, L243-gH/L243-gL1, or a fragment thereof.

6. A pharmaceutical product according to any of claims I to 5, wherein the other immunosuppressant is selected from (1) compounds which inhibit cytokine synthesis; (2) compounds which inhibit cytokine action; (3) inhibitors of DNA synthesis; or (4) inhibitors of cell maturation.

7. A pharmaceutical product according to claim 6, wherein the other immunosuppressant is selected from a cyclosporin or tacrolimus or analogues or derivatives thereof.

8. A pharmaceutical product according to any one of claims 1 to 7 additionally containing an inhibitor of B-cell function.

9. A pharmaceutical product according to claim 8 wherein said inhibitor of B-cell function is cyclophosphamide.

10. A pharmaceutical composition comprising an anti-Class II major histocompatability complex antibody, or an antigen binding fragment thereof, and at least one other, different immunosuppressant in admixture, optionally together with a pharmaceutically acceptable excipient, diluent or carrier.

11. Use of an anti-Class II major histocompatability complex antibody, or antigen binding fragment thereof, and at least one other, different immunosuppressant in the manufacture of a medicament for the suppression or elimination of rejection of a xenograft in a host.

## Patentansprüche

1. Pharmazeutisches Produkt, umfassend einen Anti-Klasse-II-Haupthistokompatibilitätskomplex-Antikörper oder ein Antigen-bindendes Fragment davon und mindestens einen anderen unterschiedlichen Immunsuppressor, zur Verwendung, um die Abstoßung eines xenogenen Transplantats in einem Wirt zu unterdrücken oder zu eliminieren.

2. Pharmazeutisches Produkt nach Anspruch 1, wobei der Anti-Klasse-II-MHC-Antikörper oder das Antigen-bindende Fragment davon zum Unterdrücken oder Eliminieren einer Wirt-T-Zellen-unabhängigen B-Zellen-Antwort befähigt ist.

3. Pharmazeutisches Produkt nach Anspruch 1 oder Anspruch 2, wobei der andere Immunsuppressor zum Unterdrücken der T-Zellenaktivierung befähigt ist.

4. Pharmazeutisches Produkt nach einem der Ansprüche 1 bis 3, wobei der Antikörper ein Anti-Human-Klasse-II-Haupthistokompatibilitätskomplex-Antikörper oder ein Antigen-bindendes Fragment davon ist.

5. Pharmazeutisches Produkt nach Anspruch 4, wobei der Antikörper L243, SFR3-DR5, L243-gH/L243-gL1 oder ein Fragment davon ist.

6. Pharmazeutisches Produkt nach einem der Ansprüche 1 bis 5, wobei der andere Immunsuppressor aus (1) Verbindungen, welche die Cytokinsynthese inhibieren, (2) Verbindungen, welche die Cytokinwirkung inhibieren, (3) Inhibitoren der DNA-Synthese oder (4) Inhibitoren der Zellreifung ausgewählt ist.

7. Pharmazeutisches Produkt nach Anspruch 6, wobei der andere Immunsuppressor aus einem Cyclosporin oder Tacrolimus oder Analogen oder Derivaten davon ausgewählt ist.

8. Pharmazeutisches Produkt nach einem der Ansprüche 1 bis 7, welches zusätzlich einen Inhibitor der B-Zellenfunktion enthält.

9. Pharmazeutisches Produkt nach Anspruch 8, wobei der Inhibitor der B-Zellenfunktion Cyclophosphamid ist.

10. Pharmazeutische Zusammensetzung, welche einen Anti-Klasse-II-Haupthistokompatibilitätskomplex-Antikörper oder ein Antigen-bindendes Fragment davon und, beigemischt mindestens einen anderen unterschiedlichen Immunsuppressor, gegebenenfalls zusammen mit einem pharmazeutisch verträglichen Bindemittel bzw. Grundmasse, Verdünnungsmittel oder Träger, umfasst.

11. Verwendung eines Anti-Klasse-II-Haupthistokompatibilitätskomplex-Antikörpers oder eines Antigen-bindenden Fragments davon und mindestens eines anderen unterschiedlichen lmmunsuppressors in der Herstellung eines Medikaments zur Unterdrückung oder Eliminierung der Abstoßung eines xenogenen Transplantats in einem Wirt.

## Revendications

1. Un produit pharmaceutique comprenant un anticorps anti-complexe majeur d'histocompatibilité de classe II, ou un fragment de liaison aux antigènes de celui-ci, et au moins un autre immunosuppresseur différent utilisé pour empêcher ou éliminer le rejet d'une xénogreffe chez un hôte.

2. Un produit pharmaceutique selon la revendication 1, dans lequel l'anticorps anti-CMH de classe II, ou un fragment de liaison aux antigènes de celui-ci, est capable d'empêcher ou d'éliminer une réponse des lymphocytes B indépendante des lymphocytes T de l'hôte.

3. Un produit pharmaceutique selon la revendication 1 ou la revendication 2, dans lequel l'autre immunosuppresseur est capable d'empêcher l'activation des lymphocytes T.

4. Un produit pharmaceutique selon l'une quelconque des revendications 1 à 3, dans lequel l'anticorps est un anticorps anti-complexe majeur d'histocompatibilité de classe II humain, ou un fragment de liaison aux antigènes de celui-ci.

5. Un produit pharmaceutique selon la revendication 4, dans lequel l'anticorps est L243, SFR3-DR5, L243-gH/L243-gL1, ou un fragment de celui-ci.

6. Un produit pharmaceutique selon l'une quelconque des revendications 1 à 5, dans lequel l'autre immunosuppresseur est sélectionné parmi (1) des composés qui inhibent la synthèse de la cytokine ; (2) des composés qui inhibent l'action de la cytokine ; (3) des inhibiteurs de la synthèse de l'ADN ; ou (4) des inhibiteurs de la maturation cellulaire.

7. Un produit pharmaceutique selon la revendication 6, dans lequel l'autre immunosuppresseur est sélectionné parmi une cyclosporine, le tracrolimus ou leurs analogues ou dérivés.

8. Un produit pharmaceutique selon l'une quelconque des revendications 1 à 7 contenant, en outre, un inhibiteur de la fonction des lymphocytes B.

9. Un produit pharmaceutique selon la revendication 8 dans lequel ledit inhibiteur de la fonction des lymphocytes B est le cyclophosphamide.

10. Une composition pharmaceutique comprenant un anticorps anti-complexe majeur d'histocompatibilité de classe II, ou un fragment de liaison aux antigènes de celui-ci, et au moins un autre immunosuppresseur différent en mélange, éventuellement avec un excipient, diluant ou véhicule pharmaceutiquement acceptable.

11. Utilisation d'un anticorps anti-complexe majeur d'histocompatibilité de classe II, ou d'un fragment de liaison aux antigènes de celui-ci, et d'au moins un autre immunosuppresseur différent, dans la fabrication d'un médicament pour l'inhibition ou l'élimination du rejet d'une xénogreffe chez un hôte.
